(19) Europäisches Patentamt
European Patent Office
Office européen des brevets

(11) Veröffentlichungsnummer: **0 011 747**
**B1**

# (12) EUROPÄISCHE PATENTSCHRIFT

(45) Veröffentlichungstag der Patentschrift: **19.05.82**

(21) Anmeldenummer: **79104388.8**

(22) Anmeldetag: **08.11.79**

(51) Int. Cl.³: **C 07 D 223/16,**
A 61 K 31/55 //C07D405/12,
C07C131/08, C07C143/68

## (54) Aminopropanolderivate des 6-Hydroxy-2,3,4,5-tetrahydro-1H-1-benzazepin-2-ons, Verfahren zu ihrer Herstellung und diese enthaltende pharmazeutische Zubereitungen.

(30) Priorität: **18.11.78 DE 2850078**

(43) Veröffentlichungstag der Anmeldung:
**11.06.80 Patentblatt 80/12**

(45) Bekanntmachung des Hinweises auf die Patenterteilung:
**19.05.82 Patentblatt 82/20**

(84) Benannte Vertragsstaaten:
**AT BE CH DE FR GB IT LU NL SE**

(56) Entgegenhaltungen:
**JOURNAL OF MEDICINAL CHEMISTRY, Band 17, Nr. 5, 1974, Columbus, Ohio, USA, KAZUYUKI NAKAGAWA et al.: "Derivatives of 3,4-dihydrocarbostyril as β-adrenergic blocking agents", Seiten 529—533**
**JOURNAL OF MEDICINAL CHEMISTRY, Band 21, Nr. 9, 1978, Columbus, Ohio, USA, MORDECHAI EREZ et al.: "Cardioselectivity as a function of molecular structure in β-adrenoceptor blocking agents of the 1-(para-substituted aryloxy)3-(isopropylamino)propan-2-ol Type", Seiten 982—984**

(73) Patentinhaber: **BASF Aktiengesellschaft**
**Carl-Bosch-Strasse 38**
**D-6700 Ludwigshafen (DE)**

(72) Erfinder: **Franke, Albrecht, Dr. Dipl.-Chem.**
**Mandelring 11**
**D-6706 Wachenheim (DE)**
Erfinder: **Lenke, Dieter, Dr.**
**Kekuleplatz 1**
**D-6700 Ludwigshafen (DE)**
Erfinder: **Gries, Josef, Dr.**
**Roemerweg 43**
**D-6706 Wachenheim (DE)**
Erfinder: **Lehmann, Hans Dieter, Dr.**
**Im Hefen 15**
**D-6945 Hirschberg-Leutershausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 747 B1

# Aminopropanolderivate des 6-Hydroxy-2,3,4,5-tetrahydro-1H-1-benzazepin-2-ons, Verfahren zu ihrer Herstellung und diese enthaltende pharmazeutische Zubereitungen

Die vorliegende Erfindung betrifft neue Aminopropanolderivate des 6-Hydroxy-2,3,4,5-tetrahydro-1H-1-benzazepin-2-ons und ihre Säureadditionssalze, sowie die Herstellung und diese Verbindungen enthaltenden pharmazeutischen Zubereitungen.

Es ist beispielsweise bekannt, daß Aminopropanolderivate des 7-Hydroxy-2,3,4,5-tetrahydro-1H-2-benzazepin-1-ons (J. Med. Chem. *16*, 516—519 (1973)), des 5-Hydroxy-3,4-dihydrocarbostyrils (J. Med. Chem. *17*, 529—533 (1974)) und des 7-Hydroxy-2,3,4,5-tetrahydro-1H-1-benzazepin-2-ons (J. Med. Chem. *21*, 982—984 (1978)) β-sympatholytische Wirkungen aufweisen.

Es wurde gefunden, daß Verbindungen der allgemeinen Formel (I),

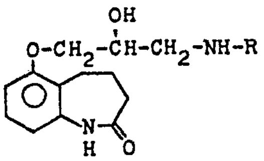

(I)

in der R einen an dem Aminostickstoff benachbarten Kohlenstoffatom verzweigten Alkylrest mit 3 bis 6 C-Atomen, 3-Butin-2-yl oder 3-Methyl-1-butin-3-yl bedeutet, und ihre physiologisch verträglichen Säureadditionssalze eine höhere pharmakologische Aktivität aufweisen.

Als verzweigte Alkylreste mit 3 bis 6 C-Atomen sind beispielsweise Isopropyl, Isobutyl, sek.-Butyl, tert.-Butyl, Pentyl-2, 2-Methyl-butyl-2, 3-Methyl-butyl-2, 3-Methyl-pentyl-3, 2,3-Dimethyl-butyl-2 zu nennen.

Als bevorzugt sind die folgenden Verbindungen hervorzuheben:

6-(2-Hydroxy-3-isopropylaminopropoxy)-2,3,4,5-tetrahydro-1H-1-benzazepin-2-on

6-(2-Hydroxy-3-sec.-butylaminopropoxy)-2,3,4,5-tetrahydro-1H-1-benzazepin-2-on

6-(2-Hydroxy-3-tert.-butylaminopropoxy)-2,3,4,5-tetrahydro-1H-1-benzazepin-2-on

6-[2-Hydroxy-3-(3-methyl-1-butin-3-ylamino)-propoxy]-2,3,4,5-tetrahydro-1H-1-benzazepin-2-on.

Die erfindungsgemäßen Verbindungen der Formel (I) können hergestellt werden, indem man ein 2,3,4,5-Tetrahydro-1H-1-benzazepin-2-on der allgemeinen Formel (II)

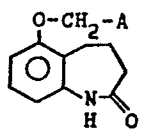

(II),

in der A den Rest

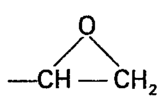 oder 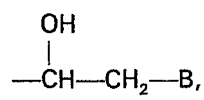

wobei B für eine nukleofuge Abgangsgruppe steht, bedeutet, mit einem Amin der allgemeinen Formel

$$H_2N—R,$$

in der R die oben angegebene Bedeutung hat, zweckmäßigerweise in einem Lösungsmittel und gegebenenfalls in Gegenwart eines säurebindenden Mittels in an sich bekannter Weise umsetzt und gegebenenfalls die erhaltenen Verbindungen in ein Säureadditionssalz einer physiologisch verträglichen Säure überführt.

Die Abgangsgruppe B stellt bevorzugt ein Halogenatom, besonders Chlor, Brom oder Jod, dar. Daneben kommen aber auch als Abgangsgruppen aliphatische oder aromatische Sulfonsäurereste, insbesondere der Methansulfonsäure-, p-Toluolsulfonsäure- und Benzolsulfonsäurerest, in Betracht.

Die Umsetzungen werden bei Raumtemperaturen oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck gegebenenfalls unter Erwärmung auf den angegebenen Temperatur-bereich durchgeführt werden.

Die Ausgangsverbindungen können direkt, d.h. ohne Zugabe eines Verdünnungs- oder Lösungsmittels, umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines Benzolkohlenwasserstoffs, wie Benzol oder eines Alkylbenzols, wie Toluol oder Xylol, oder eines aliphatischen Kohlenwasserstoffes, wie Hexan, Heptan oder

Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Auch ist das in überschüssiger Menge verwendete Amin der Formel $H_2N—R$ gegebenenfalls als Verdünnungs- oder Lösungsmittel geeignet.

Bevorzugte Lösungsmittel bei der Umsetzung von 6-(2,3-Epoxypropoxy)-2,3,4,5-tetrahydro-1H-1-benzazepin-2-on mit einem Amin $R—NH_2$ sind niedere Alkohole, insbesonders Äthanol oder Isopropanol, wobei die Umsetzung bevorzugt bei Temperaturen von 50 bis 100°C und bei Normaldruck durchgeführt wird.

Bei der nukleophilen Substitution eines Restes B sind als Lösungsmittel ein niederes aliphatisches Keton, insbesondere Aceton oder Methyl-isopropylketon, ein cyclischer Äther, insbesondere Tetrahydrofuran oder Dioxan, oder ein Dialkylformamid, wie Dimethylformamid, und Temperaturen von 90 bis 120°C bevorzugt. Gegebenenfalls ist die Gegenwart einer katalytischen Menge Natrium- oder Kaliumjodid zweckmäßig.

Es sei erwähnt, daß als Ausgangsverbindung der Formel (II) gegebenenfalls auch eine Mischung des Epoxids mit einem Halogenhydrin in Betracht kommt.

Bei einer zweckmäßigen Ausführungsform zur nukleophilen Substitution des Restes B durch das verwendete Amin wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen inbesondere die des Natriums und Kaliums in Betracht. Dabei wird die Base in stöchiometrischer Menge oder in geringem Überschuß verwendet. Gegebenenfalls ist es zweckmäßig, das zur Umsetzung verwendete Amin $H_2N—R$ in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung des 6-Hydroxy-2,3,4,5-tetrahydro-1H-1-benzazepin-2-ons (III)

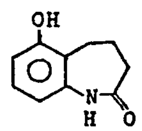

(III)

mit einem Epihalogenhydrin oder einem $\alpha,\omega$-Diahalogen-2-propanol erhalten werden. Als Epihalogenhydrin kommen Epichlorhydrin, Epibromhydrin und Epijodhydrin und als $\alpha,\omega$-Dihalogen-2-propanol kommen insbesondere 1,3-Dichlor- und 1,3-Dibrom-2-propanol in Betracht.

Die Umsetzungen des 6-Hydroxy-2,3,4,5-tetrahydro-1H-1-benzazepin-2-ons zur Herstellung der Ausgangsverbindungen der Formel (II) werden zweckmäßigerweise bei Temperaturen von 0 bis 120°C und unter Normaldruck oder in einem geschlossenen Gefäß unter erhöhten Drucken durchgeführt. Die Umsetzungen werden zweckmäßigerweise in einem inerten Verdünnungs- oder Lösungsmittel, z.B. einem niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem aliphatischen oder cyclischen Äther, wie Dialkyläther, Tetrahydrofuran oder Dioxan, einem Dialkylformamid, wie Dimethylformamid oder Diäthylformamid, oder Dimethylsulfoxid oder Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuß verwendet werden.

Bevorzugt wird das 6-Hydroxy-2,3,4,5-tetrahydro-1H-1-benzazepin-2-on mit Epibromhydrin oder 1,2-Dibrompropanol-2 in einem Lösungsmittelgemisch aus einem Äther und einem polaren aprotischen Lösungsmittel, insbesondere Tetrahydrofuran und Hexamethylphosphortriamid, bei Temperaturen zwischen 0 und 50°C oder in Aceton bei Siedetemperatur umgesetzt.

Das als Ausgangsverbindung benötigte 6-Hydroxy-2,3,4,5-tetrahydro-1H-1-benzazepin-2-on (III) läßt sich durch Ätherspaltung eines 6-Alkoxy-2,3,4,5-tetrahydro-1H-1-benzazepin-2-ons der allgemeinen Formel (IV),

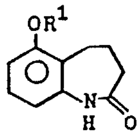

(IV)

in der $R^1$ einen Alkylrest mit 1 bis 4 C-Atomen oder einen $\alpha$-Aralkylrest bedeutet, herstellen.

Als Alkylrest für $R^1$ seien der Methyl-, Äthyl-, Propyl- und Butylrest genannt, davon ist der Methylrest bevorzugt. Der bevorzugte $\alpha$-Aralkylrest ist der Benzylrest.

Die Ätherspaltung wird in an sich bekannter Weise mit den für Ätherspaltungen bekannten und üblichen Reagenzien durchgeführt. Besonders hervorzuhebende Spaltungsreagenzien sind Halogenwasserstoffsäuren, bevorzugt wäßrige Brom- und Jodwasserstoffsäure, gegebenenfalls in Gegenwart von rotem Phosphor und/oder aliphatischen Carbonsäuren mit 1 bis 5 C-Atomen, vorzugsweise Ameisensäure oder Essigsäure als Verdünnungsmittel, oder Pyridinhydrohalogenide, beispielsweise Pyridiniumchlorid oder -bromid, oder Lithiumjodid in Collidin oder Diboran oder Bortrihalogenide, vorzugsweise Bortribromid, in einem aromatischen Kohlenwasserstoff, wie Benzol, Toluol oder Xylol, oder Aluminiumchlorid in einem geeigneten Lösungsmittel, wie beispielsweise Schwefelkohlenstoff oder Dimethylformamid.

Die Ätherspaltungsreaktionen können bei Raumtemperatur oder erhöhten Temperaturen, beispielsweise Temperaturen von 100 bis 150°C, bei Normaldruck oder in einem geschlossenen Gefäß bei erhöhtem Druck durchgeführt werden. Die Reaktionen können in einem inerten Lösungsmittel oder aber in der Schmelze des die Ätherspaltung bewirkenden Stoffes, beispielsweise in einer Pyridinhalogenidschmelze oder $AlCl_3$/DMF-Schmelze, durchgeführt werden.

Die Reaktionszeit ist von der Reaktionstemperatur und dem zur Atherspaltung angewandten Reagenz abhängig; im allgemeinen sind die Spaltungen nach 5 Stunden beendet.

Der Benzylrest wird darüber hinaus bevorzugt hydrogenolytisch in Gegenwart eines Katalysators, wie z.B. Palladium auf einem Träger, wie Kohlenstoff, Aluminiumoxid oder Keiselgur, in einem geeigneten Lösungsmittel, wie z.B. Methanol, Athanol oder Propanol, abgespalten.

Die Verbindungen der Formel (IV), von denen das 6-Methoxy-2,3,4,5-tetrahydro-1H-1-benzazepin-2-on für die Herstellung von 6-Hydroxy-2,3,4,5-tetrahydro-1H-1-benzazepin-2-on (III) besonders beeignet ist, sind zugänglich nach an sich bekannten Methoden durch Ringerweiterung der entsprechenden 5-Alkoxy-1-tetralone. Als Ringerweiterungsreaktionen kommen die Schmidt-Reaktion und die Beckmann-Umlagerung in Betracht, wie sie in Houben-Weyl, Bd. 11/2, S. 546—554 (Georg Thieme Verlag, Stuttgart; 1958) beschrieben sind. Speziell wird die Schmidt-Reaktion am 1-Tetralon in J. Chem. Soc. *1937*, 456 ff und die Beckmann-Umlagerung über das Oxim-benzosulfonat des 1-Tetralons in Liebigs Annalen *586*, 30 ff (1954) ausführlich beschrieben.

Die in untergeordnetem Maße sich bildenden isomeren 6-Alkoxy-2,3,4,5-tetrahydro-1H-2-benzazepin-1-one lassen sich ohne Schwierigkeiten durch Umkristallisation abtrennen. Die Struktur der erhaltenen 6-Alkoxy-2,3,4,5-tetrahydro-1H-1-benzazepin-2-one wird NMR-spektroskopisch ermittelt (s. dazu auch J. Med. Chem. *16*, 516—519 (1973)).

Es sei darauf hingewiesen, daß sich das 6-Hydroxy-2,3,4,5-tetrahydro-1H-1-benzazepin-2-on auch direkt aus 6-Hydroxy-tetralon durch die Schmidt-Reaktion durch Ringerweiterung herstellen läßt, wie es für das 7-Hydroxy-2,3,4,5-tetrahydro-1H-1-benzazepin-2-on beschrieben wird (J. Med. Chem. *16*, 516—519 (1973)).

Die erfindungsgemäßen Verbindungen der Formel (I) besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Brom-campher-8-sulfonsäure, in die optisch aktiven Antipoden getrennt werden können.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure, und als organische Säuren beispielsweise Oxalsäure, Maleinsäure, Fumarsäure, Milchsäure, Weinsäure, Äpfelsäure, Zitronensäure, Salicylsäure, Adipinsäure oder Benzolsäure oder können aus Fortschritte der Arzneimittelforschung, Band *10*, Seiten 224 bis 225, Birkhäuser Verlag, Basel und Stuttgart, 1966, oder dem Journal of Pharmaceuticals Sciences, Volume 66, Seiten 1 bis 5 (1977), entnommen werden.

Die Säureadditionssalze werden in der Regel in an sich bekannter Weise durch Mischung der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung

können auch Mischungen der genannten Lösungsmittel verwendet werden. Darüber hinaus können pharmazeutisch vertretbare wäßrige Lösungen von Säure-Additionsverbindungen der Aminopropanol-Derivate der allgemeinen Formel (I) durch Auflösen der freien Basen der allgemeinen Formel (I) in einer wäßrigen Säurelösung hergestellt werden.

Die erfindungsgemäßen Verbindungen der Formel I und ihre physiologisch verträglichen Säureadditionssalze weisen wertvolle pharmakologische Eigenschaften auf und können bei Herz- und Kreislauferkrankungen verwendet werden. Aufgrund ihrer beta-sympatholytischen Wirkung eignen sie sich besonders zur Behandlung der coronaren Herzkrankheit, von Herzrhythmusstörungen und der Hypertonie.

Ihre hohe β-sympatholytische Aktivität übertrifft beispielsweise die der bekannten Verbindung Propranolol bei weitem. Dieser Befund ist deshalb überraschend und war nicht vorhersehbar, da die zu (I) isomeren 7-(2-Hydroxy-3-alkylaminopropoxy)-2,3,4,5-tetrahydro-1H-2-benzazepin-1-one (J. Med. Chem. *16*, 516—519 (1973)), Verbindungen, in denen im Vergleich zu den erfindungsgemäßen Verbindungen die (2-Hydroxy-3-alkylamino-propoxy)-gruppe sich in 7-Stellung befindet und zugleich noch im Benzazepinring die NH— und C=O-Gruppe untereinander vertauscht sind, nur sehr schwache β-Sympatholytika darstellen. Auch die zu (I) ähnlichen 5-(2-Hydroxy-3-alkylamino-propoxy)-3,4-dihydrocarbostyrile (J. Med. Chem. *17*, 529—533 (1974)) erreichen nicht die Aktivität der erfindungsgemäßen Verbindungen.

Die β-sympatholytische Wirkung wurde an Katzen und Hunden getestet. Als Vergleichssubstanz diente das bekannte β-Sympatholytikum Propranolol. Zur Prüfung wurde das Modell der Isoproterenol-Tachycardie verwendet.

Isoproterenol (1 μg/kg i.v.) verursacht an der mit Hexobarbital (200 mg/kg i.m.) narkotisierten Katze (männlich und weiblich, mischrassig, Gewicht 1,7 bis 4,0 kg) Herzfrequenzsteigerungen um durchschnittlich 61 $\pm$ 2,4 Schläge/min. β-Sympatholytika hemmen diese Tachycardie. Isoproterenol wurde vor und 10 min nach der i.v. bzw. 30 min nach der intraduodenalen Applikation der Prüfsubstanzen injiziert. Es werden die Dosen bestimmt, welche die Isoproterenol-Tachycardie um 50 bis 60% hemmen.

Am wachen Hund führt Isoproterenol (1 μg/kg i.v.) zu einer Steigerung der Herzfrequenz um etwa 100 Schläge/min. β-Sympatholytika hemmen diese Tachycardie. Isoproterenol wurde vor sowie 10 min nach der i.v. Zufuhr der Prüfsubstanzen appliziert.

Zwischen den Logarithmen der applizierten Dosen (mg/kg) der Prüfsubstanzen und der Hemmung der Isoproterenol-Tachycardie (%) bestehen lineare Beziehungen. Aus diesen Beziehungen werden als ED 50% die Dosen ermittelt, welche die Isoproterenol-Tachycardie um 50% hemmen.

Außer der β-sympatholytischen Wirkung wurde die akute Toxizität an Gruppen von je 10 weiblichen NMRI-Mäusen, Gewicht 22—27 g, bei intraperitonealer Applikation ermittelt. Als LD 50 wurde die Dosis berechnet (Probit-Analyse) nach der 50% der Tiere innerhalb von 24 Stunden starben.

Die erfindungsgemäßen Verbindungen sind β-sympatholytisch hochwirksam. Aus der Tabelle 1 geht hervor, daß die für eine 50 bis 60 %ige Hemmung der Isoproterenol-Tachycardie erforderlichen Dosen bei der pharmakotherapeutisch wichtigen enteralen (intraduodenalen) Applikation an der Katze 2- (Beispiel 1) bzw. 4,7-mal (Beispiel 2) kleiner sind, als die entsprechende Dosis von Propranolol.

Bei i.v. Gabe werden 4,7-mal niedrigere (Beispiel 2) bzw. 2-mal höhere (Beispiel 1) Dosen benötigt als von Propranolol.

Die hohe β-sympatholytische Wirksamkeit von Beispiel 2 ist auch am Hund festzustellen. Hier wurde eine ED 50 von 0,0043 mg/kg ermittelt. Damit ist die Substanz 24-mal wirksamer als Propranolol (ED 50% = 0,10 mg/kg).

Die Toxizität von Beispiel 2 ist geringer als die von Propranolol. Die LD 50 bei intraperitonealer Applikation an der Maus beträgt 237 mg/kg, die von Propranolol 108 mg/kg.

TABELLE 1: β-sympatholytische Wirkung an der Katze

| | Isoproterenol-Tachycardie | | | |
|---|---|---|---|---|
| | Appl. i.v. | | Appl. i.d. | |
| Beispiel Nr. | mg/kg | % 1) | mg/kg | % 1) |
| 1 | 0,215 | 59 | 0,464 | 56 |
| 2 | 0,0215 | 57 | 0,215 | 52 |
| Propranolol | 0,1 | 52 | 1,0 | 57 |

1) % Hemmung.

Gegenstand der vorliegenden Erfindung sind demnach auch therapeutische Mittel oder Zubereitungen, die neben üblichen Träger- und Verdünnungsmitteln eine Verbindung der Formel I als Wirkstoff enthalten, sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Film-tabletten, Dragees, Kapseln, Pillen, Pulver, Lösungen oder Suspensionen oder Depotformen. Dabei ist die orale Applikation bevorzugt.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmitteln, wie Maisstärke oder Alginsäure, Bindemitteln, wie Stärke oder Gelatine, Gleitmitteln, wie Magnesiumstearat oder Talk, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Dragee-überzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Saccharin, Cyclamat oder Zucker sowie z.B. Aromastoffe, wie Vanillin oder Orangenextrakt, enthalten. Sie können außerdem Suspendierhilfsstoffe, wie Natriumcarboxymethylcellulose, oder Konservierungsstoffe, wie p-Hydroxybenzoate, enthalten. Wirkstoffe enthaltende Kapseln, können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln, wie Neutralfetten oder Polyäthylenglykol bzw. deren Derivaten, herstellen.

Als Einzeldosis der erfindungsgemäßen Verbindungen kommen 0,5 bis 50 mg, vorzugsweise 1 bis 10 mg, am Menschen in Betracht.

Die vorliegende Erfindung wird durch die nachfolgenden Ausführungsbeispiele näher erläutert:

Herstellung von Ausgangsverbindungen

Beispiel Ia

6-Hydroxy-2,3,4,5-tetrahydro-1H-1-benzazepin-2-on ($AlCl_3$/Dimethylformamid-Spaltung)

65 g wasserfreies Aluminiumchlorid werden unter heftigem Rühren und eventuellem Kühlen mit 10 ml DMF versetzt. Zu der entstandenen Schmelz werden 13,5 g (0,07 Mol) 6-Methoxy-2,3,4,5-tetrahydro-1H-1-benzazepin-2-on portionsweise eingetragen und dann der Kolbeninhalt auf 110 bis

140°C erhitzt, 10 Minuten wird bei dieser Temperatur belassen und anschließend rührt man ohne weitere Heizung 30 Minuten nach. Der Kolbeninhalt wird auf Eiswasser gegossen, der gebildete sandfarbene Niederschlag abgesaugt und das Filtrat einige Male mit Äther nachextrahiert, die Ätherphasen vereinigt, getrocknet und einrotiert. Die vereinigten Rückstände werden unter Zusatz von Tierkohle aus Aceton/Cyclohexan/Essigester umkristallisiert. Man erhält 6,3 g 6-Hydroxy-2,3,4,5-tetrahydro-1H-1-benzazepin-2-on (51% Ausbeute) vom Fp. 244—245°C.

$C_{10}H_{11}NO_2$ (177,2)

| | | | |
|---|---|---|---|
| ber. | C 68,7% | H 7,3% | N 7,3% |
| gef. | C 68,5% | H 7,1% | N 7,2% |

Beispiel Ib

6-Hydroxy-2,3,4,5-tetrahydro-1H-1-benzazepin-2-on (Pyridiniumchlorid-Spaltung)

3,8 g (0,02 Mol) 6-Methoxy-2,3,4,5-tetrahydro-1H-1-benzazepin-2-on und 10 g Pyridiniumchlorid werden 2 Stunden lang auf 200 bis 220°C erhitzt. Die Schmelze wird abgekühlt, auf Wasser gegossen, mit 2 n-$H_2SO_4$ angesäuert und mit Äther mehrere Male extrahiert. Die organische Phase wird getrocknet, eingeengt und der Rückstand unter Zusatz von Tierkohle aus Aceton/Cyclohexan/Essigester umkristallisiert. Man erhält 1,6 g 6-Hydroxy-2,3,4,5-tetrahydro-1H-1-benzazepin-2-on vom Fp. 243 bis 244°C. Identisch mit dem unter Ia erhaltenen Produkt.

Beispiel II

6-Methoxy-2,3,4,5-tetrahydro-1H-1-benzazepin-2-on

9,7 g (0,029 Mol) 5-Methoxy-tetralon-1-oximbenzolsulfonat werden in 800 bis 900 ml 50%iger Essigsäure bis zur vollständigen Lösung (ca. 60 Min.) auf dem Wasserbad gehalten. Anschließend wird mit dem doppelten Volumen Wasser verdünnt und mit Ather mehrere Male extrahiert. Die vereinigten Atherextrakte werden zuerst mit wäßriger Bicarbonatlösung, dann mehrere Male mit Wasser gewaschen, getrocknet und eingeengt. Der verbleibende Rückstand kristallisiert beim Stehenlassen aus und ist analysenrein. Es werden 4,2 g 6-Methoxy-2,3,4,5-tetrahydro-1H-1-benzazepin-2-on (75,7% Ausbeute) vom Fp. 162°C erhalten.

$C_{11}H_{13}NO_2$ (191,2)

| | | | |
|---|---|---|---|
| ber.: | C 69,0% | H 6,8% | N 7,3% |
| gef.: | C 68,8% | H 6,9% | N 7,2% |

Beispiel III

5-Methoxy-tetralon-1-oxim-benzolsulfonat

10 g (0,042 Mol) Tetralon-1-oxim werden in 80 ml wasserfreiem Pyridin gelöst. Bei Raumtemperatur werden 10,8 g Benzolsulfonsäurechlorid in 15 Min. zugetropft und die Lösung 12 Stunden stehen gelassen. Dann werden 5 ml Wasser zugesetzt und anschließend die Lösung in 300 ml eiskalte 4 n-HCl gegossen. Der entstandene Niederschlag wird abfiltriert, getrocknet und aus Äthanol umkristallisierte. Es wurden 13,6 g (92,8% Ausbeute) 5-Methoxy-tetralon-1-oxim-benzolsulfonat vom Fp. 142—144°C isoliert.

$C_{17}H_{17}NO_4S$ (331):

| | | | | |
|---|---|---|---|---|
| ber.: | C 61,6% | H 5,2% | N 4,2% | S 9,6% |
| gef.: | C 61,7% | H 5,3% | N 4,3% | S 9,7% |

Beispiel IV

5-Methoxy-tetralon-1-oxim

17,7 g (0,1 Mol) käufliches 5-Methoxy-1-tetralon (Hersteller Firma Aldrich) werden zusammen mit 18,4 g (0,26 Mol) Hydroxylaminhydrochlorid und 22,6 g Natriumhydrogencarbonat (0,26 Mol) in 450 ml Methanol und 80 ml Wasser 36 Stunden unter Rückfluß gehalten. Dann wird das Lösungsmittel am Rotationsverdampfer entfernt, der Rückstand mit Wasser ausgerührt und die Ausfällung abgesaugt, getrocknet und aus Toluol umkristallisiert. Es werden 15,2 g 5-Methoxy-1-tetralon-oxim vom Fp. 158—159°C erhalten (79,6% Ausbeute).

$C_{11}H_{13}NO_2$ (191,2)

| | | | |
|---|---|---|---|
| ber.: | C 69,0% | H 6,8% | N 7,3% |
| gef.: | C 69,1% | H 6,6% | N 7,1% |

Beispiel V

6-(2,3-Epoxy-propoxy)-2,3,4,5-tetrahydro-1H-1-benzazepin-2-on

5,3 g (0,03 Mol) 6-Hydroxy-2,3,4,5-tetrahydro-1H-benzazepin-2-on werden zusammen mit 5 ml Epibromhydrin und 4,5 g Kaliumcarbonat in 250 ml Methylisobutylketon 48 Stunden am Rückfluß gehalten. Nach dem Abkühlen wird abfiltriert und das Filtrat am Rotationsverdampfer unter vermindertem

Druck eingeengt. Der Rückstand wird unter Zusatz von Tierkohle aus Cyclohexan umkristallisiert. Es werden 4,2 g (60% Ausbeute) 6-(2,3-Epoxy-propoxy)-2,3,4,5-tetrahydro-1H-1-benzazepin-2-on vom Fp. 121—123°C erhalten.

$C_{13}H_{15}NO_3$ (233)

| | | | |
|---|---|---|---|
| ber.: | C 66,9% | H 6,5% | N 6,0% |
| gef.: | C 66,6% | H 6,6% | N 5,8% |

Herstellung der erfindungsgemäßen Verbindungen.

Beispiel 1

6-(2-Hydroxy-3-isopropylaminopropoxy)-2,3,4,5-tetrahydro-1H-1-benzazepin-2-on

3,3 g (0,014 Mol) 6-(2,3-Epoxypropoxy)-2,3,4,5-tetrahydro-1H-1-benzazepin-2-on werden in 400 ml n-Propanol gelöst, mit 3 g Isopropylamin versetzt und 4 bis 6 Stunden auf dem Wasserbad gehalten. Anschließend wird das Lösungsmittel und überschüssiges Amin am Rotationsverdampfer abdestilliert, zweimal mit Methanol aufgenommen und wieder abdestilliert. Der Rückstand wird über eine Kieselgelsäule chromatographiert (Methanol als Laufmittel). Es werden 1,65 g 6-(2-Hydroxy-3-isopropylaminopropoxy)-2,3,4,5-tetrahydro-1H-1-benzazepin-2-on vom Fp. 143—145°C erhalten; 39,9% Ausbeute.

$C_{16}H_{24}N_2O_3$ (292)

| | | | |
|---|---|---|---|
| ber.: | C 65,7% | H 8,3% | N 9,6% |
| gef.: | C 65,4% | H 8,4% | N 9,4% |

Beispiel 2

6-(2-Hydroxy-3-tert.-butylaminopropoxy)-2,3,4,5-tetrahydro-1H-1-benzazepin-2-on-hydrochlorid

Analog Beispiel 1 werden 9,4 g (0,04 Mol) 6-(2,3-Epoxypropoxy)-2,3,4,5-tetrahydro-1H-1-benzazepin-2-on mit 5,8 g tert.-Butylamin umgesetzt. Die Verbindung wird aus Äthanol/Aceton mit ätherischer HCl als Hydrochlorid gefällt und dieses aus Athanol/Aceton/Ather umkristallisiert. Es werden 3,6 g 6-(2-Hydroxy-3-tert.-butylaminopropoxy)-2,3,4,5-tetrahydro-1H-1-benzazepin-2-on Hydrochlorid vom Fp. 202°C erhalten; 26,2% Ausbeute.

$C_{17}H_{27}N_2O_3Cl$ (342,5)

| | | | | |
|---|---|---|---|---|
| ber.: | C 59,6% | H 7,9% | N 8,2% | Cl 10,4% |
| gef.: | C 59,6% | H 8,2% | N 7,6% | Cl 10,1% |

Beispiel 3

6-(2-Hydroxy-3-sec.-butylaminopropoxy)-2,3,4,5-tetrahydro-1H-1-benzazepin-2-on-hydrochlorid

Aus 1,7 g (0,007 Mol) 6-(2,3-Epoxypropoxy)-2,3,4,5-tetrahydro-1H-1-benzazepin-2-on und 2,5 g 2-Amino-butan analog Beispiel 1 und Isolierung als Hydrochlorid analog Beispiel 2. Es werden 1,05 g 6-(2-Hydroxy-3-sec.-butylaminopropoxy)-2,3,4,5-tetrahydro-1H-1-benzazepin-2-on-hydrochlorid vom Fp. 177°C erhalten; 42% Ausbeute.

$C_{17}H_{27}N_2O_3Cl$ (342,5)

| | | | | |
|---|---|---|---|---|
| ber.: | C 59,6% | H 7,9% | N 8,2% | Cl 10,4% |
| gef.: | C 59,3% | H 8,1% | N 7,7% | Cl 10,2% |

Beispiel 4

6-[2-Hydroxy-3-(3-methyl-1-butin-3-ylamino)-propoxy]-2,3,4,5-tetrahydro-1H-1-benzazepin-2-on-hydrochlorid

Aus 1,7 g (0,007 Mol) 6-(2,3-Epoxypropoxy)-2,3,4,5-tetrahydro-1H-1-benzazepin-2-on und 1,25 g 3-Amino-3-methyl-1-butin werden analog Beispiel 1 umgesetzt. Es werden 0,85 g 6-[2-Hydroxy-3(3-methyl-1-butin-3-ylamino)-propoxy]-2,3,4,5-tetrahydro-1H-1-benzazepin-2-on-hydrochlorid vom Fp. 177°C erhalten; 33% Ausbeute.

$C_{18}H_{25}N_2O_3Cl$ (352,5)

| | | | | |
|---|---|---|---|---|
| ber.: | C 61,3% | H 7,1% | N 7,9% | Cl 10,1% |
| gef.: | C 61,2% | H 7,3% | N 7,7% | Cl 10,3% |

Formulierungsbeispiele, die in üblicher Weise hergestellt werden:

1. *Tabletten*

a)

| | |
|---|---|
| Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

b)

| | |
|---|---|
| Ein Wirkstoff der Formel I | 10 mg |
| Lactose | 188 mg |
| Avicel | 80 mg |
| Polywachs 6000 | 20 mg |
| Magnesiumstearat | 2 mg |
| | 300 mg |

c)

| | |
|---|---|
| Eine Verbindung der Formel I | 10 mg |
| Polyvinylpyrrolidon (mittl. M.G. 25 000) | 210 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und Magnesiumstearat vermischt und zu Tabletten a 280 mg verpreßt.

2. *Beispiel für Dragees*

| | |
|---|---|
| Eine Verbindung der Formel I | 2,5 mg |
| Lactose | 90,5 mg |
| Maisstärke | 60 mg |
| Polyvinylpyrrolidon | 6 mg |
| Magnesiumstearat | 1 mg |
| | 160,0 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50°C getrocknet und nochmals

durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. *Kapselformulierung*

| | |
|---|---|
| Eine Verbindung der Formel I | 5,0 mg |
| Magnesiumstearat | 2,0 mg |
| Milchzucker | 19,3 mg |

4. *Injektionslösung*

| | |
|---|---|
| Eine verbindung der Formel I | 1,0 mg |
| Natriumchlorid | 9 mg |
| destilliertes Wasser, q.s. auf 1,0 ml | |

**Patentansprüche für die Vertragsstaten: BE, CH, DE, FR, GB, IT, LU, NL und SE**

1. Verbindungen der allgemeinen Formel (I),

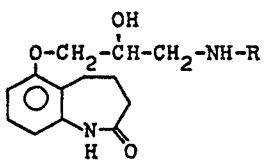

(I)

in der R einen an dem Aminostickstoff benachbarten Kohlenstoffatom verzweigten Alkylrest mit 3 bit 6 C-Atomen, 3-Butin-2-yl oder 3-Methyl-1-butin-3-yl bedeutet, und ihre physiologisch verträglichen Säureadditionssalze.

2. 6-(2-Hydroxy-3-isopropylaminopropoxy)-2,3,4,5-tetrahydro-1H-1-benzazepin-2-on.

3. 6-(2-Hydroxy-3-tert.-butylaminopropoxy)-2,3,4,5-tetrahydro-1H-1-benzazepin-2-on.

4. Verbindung der Formel I nach Ansprüchen 1 bis 3 zur Verwendung als Arzneimittel bei der Behandlung der coronaren Herzkrankheit, von Herzrhythmusstörungen und der Hypertonie.

5. Verfahren zur Herstellung von Verbindungen der Formel I nach Ansprüchen 1—3, dadurch gekennzeichnet, daß man ein 2,3,4,5-Tetrahydro-1H-1-benzazepin-2-on der allgemeinen Formel (II)

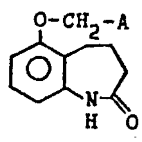

(II),

in der A den Rest

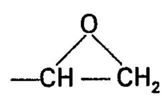 oder 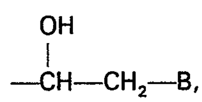

wobei B für eine nukleofuge Abgangsgruppe steht, bedeutet, mit einem Amin der allgemeinen Formel

$$H_2N—R,$$

in der R die oben angegebene Bedeutung hat, zweckmäßigerweise in einem Lösungsmittel und gegebenenfalls in Gegenwart eines säurebindenden Mittels in an sich bekannter Weise umsetzt und gegebenenfalls die erhaltenen Verbindungen in ein Säureadditionssalz einer physiologisch verträglichen Säure überführt.

6. Therapeutisches Mittel, gekennzeichnet durch einen Gehalt an einer Verbindung der Formel I nach Ansprüchen 1 bis 3 oder ihres physiologisch verträglichen Säureadditionssalzes als Wirkstoff neben üblichen Trägerstoffen und Verdünnungsmitteln.

**Patentanspruch für den Vertragsstaat: AT**

Verfahren zur Herstellung von Verbindungen der allgemeinen Formel (I),

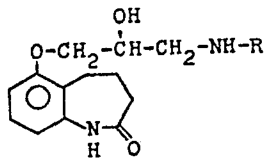

(I)

in der R einen an dem Aminostickstoff benachbarten Kohlenstoffatom verzweigten Alkylrest mit 3 bis 6 C-Atomen, 3-Butin-2-yl oder 3-Methyl-1-butin-3-yl bedeutet, und ihre physiologisch verträglichen Säureadditionssalze, dadurch gekennzeichnet, daß man ein 2,3,4,5-Tetrahydro-1H-1-benzazepin-2-on der allgemeinen Formel (II)

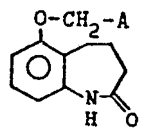

(II),

in der A den Rest

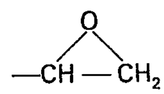 oder 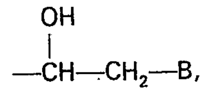

wobei B für eine nukleofuge Abgangsgruppe steht, bedeutet, mit einem Amin der allgemeinen Formel

$$H_2N—R,$$

in der R die oben angegebene Bedeutung hat, zweckmäßigerweise in einem Lösungsmittel und gegebenenfalls in Gegenwart eines säurebindenden Mittels in an sich bekannter Weise umsetzt und gegebenenfalls die erhaltenen Verbindungen in ein Säureadditionssalz einer physiologisch verträglichen Säure überführt.

**Revendications pour les Etats contractants: BE, CH, DE, FR, GB, IT, LU, NL, SE**

1. Composés de formule générale (I),

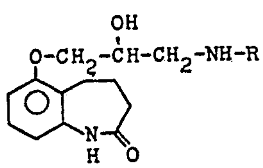

(I)

dans laquelle R représente un reste alkyle à 3 à 6 atomes C, ramifié sur l'atome de carbone voisin de l'aminoazote, 3-butin-2-yle ou 3-méthyle-1-butin-3-yle et leurs sels d'addition d'acides compatibles physiologiquement.

2. 6-(2-hydroxy-3-isopropylaminopropoxy)-2,3,4,5-tétrahydro-1H-1-benzazépin-2-one.

3. 6-(2-hydroxy-3-tert.-butylaminopropoxy)-2,3,4,5-tétrahydro-1H-1-benzazépin-2-one.

4. Composé de formule I selon les revendications 1 à 3, pour utilisation comme médicament pour le traitement des maladies cardiaques coronariennes, des troubles du rythme cardiaque et de l'hypertonie.

5. Procédé de préparation de composés de formule I selon les revendications 1—3, caractérisé par le fait que l'on fait réagir, de manière connue en soi, une 2,3,4,5-tétrahydro-1H-1-benzazépin-2-one de formule générale (II)

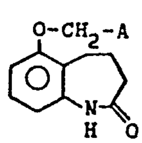

(II),

dans laquelle A représente le reste

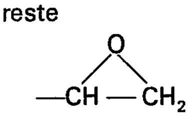 ou 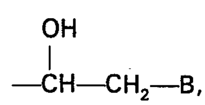

B représentant un groupe de départ nucléofuge, avec une amine de formule générale

$$H_2N—R,$$

dans laquelle R a la signification indiquée plus haut, de préférence dans un solvant et éventuellement en présence d'un agent liant les acides, et l'on transforme éventuellement les composés obtenus en un sel d'addition d'acide d'un acide compatible physiologiquement.

6. Agent thérapeutique, caractérisé par une teneur en un composé de formule I selon les revendications 1 à 3 ou son sel d'addition d'acide compatible physiologiquement, en tant que principe actif, à côté de véhicules et de diluants usuels.

**Revendication pour l'Etat contractant: AT**

Procédé de préparation de composés de formule générale (I)

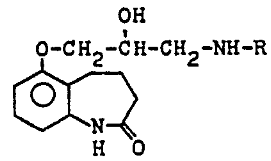

(I)

dans laquelle R représente un reste alkyle à 3 à 6 atomes C, ramifié sur l'atome de carbone voisin de l'aminoazote, 3-butin-2-yle ou 3-méthyl-1-butin-3-yle et leurs sels d'addition d'acides compatibles physiologiquement, caractérisé par le fait que l'on fait réagir, de manière connue en soi, une 2,3,4,5-tétrahydro-1H-1-benzazépin-2-one de formule générale (II)

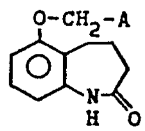

(II),

dans laquelle A représente le reste

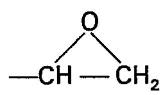

ou

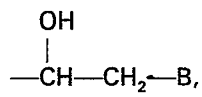

B représentant un groupe de départ nucléofuge, avec une amine de formule générale

$$H_2N—R,$$

dans laquelle R a la signification indiquée plus haut, de préférence dans un solvant et éventuellement en présence d'un agent liant les acides, et l'on transforme éventuellement les composés obtenus en un sel d'addition d'un acide compatible physiologiquement.

**Claims for the Contracting States: BE, CH, DE, FR, GB, IT, LU, NL, SE**

1. A compound of the general formula (I)

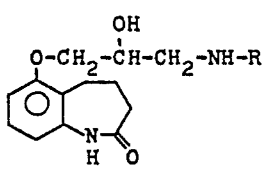

(I)

where R is alkyl of 3 to 6 carbon atoms, which is branched at the carbon adjacent to the amino nitrogen, but-3-yn-2-yl or 3-methyl-but-1-yn-2-yl, and its physiologically acceptable addition salts with acids.

2. 6-(2-Hydroxy-3-isopropylaminopropoxy)-2,3,4,5-tetrahydro-1H-1-benzazepin-2-one.

3. 6-(2-Hydroxy-3-tert.-butylaminopropoxy)-2,3,4,5-tetrahydro-1H-1-benzazepin-2-one.

4. A compound of the formula I as claimed in claims 1 to 3 for use as a drug in the treatment of coronary cardiac disorders, cardiac arrhythmias and hypertonia.

5. A process for the preparation of a compound of the formula I as claimed in claims 1 to 3, characterized in that a 2,3,4,5-tetrahydro-1H-1-benzazepin-2-one of the general formula (II)

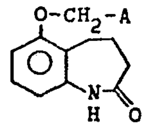

(II)

where A is

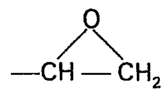 or 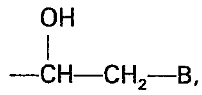

B being a nucleofugic leaving group, is reacted, in the conventional manner, with an amine of the general formula

$$H_2N—R$$

where R has the above meanings, advantageously in a solvent, and in the presence or absence of an acid-binding agent, after which the resulting compound may or may not be converted into an addition salt with a physiologically acceptable acid.

6. A therapeutic agent characterized by a content of a compound of the formula I as claimed in claims 1 to 3 or a physiologically acceptable addition salt thereof with an acid, as the active ingredient in addition to conventional carriers and diluents.

**Claim for the Contracting State: AT**

A process for the preparation of a compound of the general formula (I)

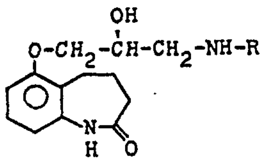

(I)

where R is alkyl of 3 to 6 carbon atoms, which is branched at the carbon adjacent to the amino nitrogen, but-3-yl-2-yl or 3-methyl-but-1-yn-2-yl, and its physiologically acceptable addition salts with acids, characterized in that a 2,3,4,5-tetrahydro-1H-1-benzazepin-2-one of the general formula (II)

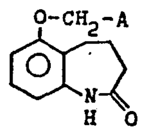

(II)

where A is

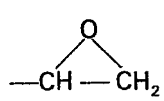 or 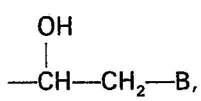

B being a nucleofugic leaving group, is reacted, in the conventional manner, with an amine of the general formula

$$H_2N—R$$

where R has the above meanings, advantageously in a solvent, and in the presence or absence of an acid-binding agent, after which the resulting compound may or may not be converted into an addition salt with a physiologically acceptable acid.